# EUROPEAN PATENT APPLICATION

(11) **EP 4 738 646 A1**
(43) Date of publication of application: **06.05.2026**
(21) Application number: 24849567.3
(22) Date of filing: 30.07.2024
(51) Int. Cl.: H02J 50/00, H02J 50/80, H02J 50/90, H01F 7/02, H05K 1/18, G06F 1/16

(54) **ELECTRONIC DEVICE FOR CHARGING WEARABLE DEVICE**

(30) Priority: 02.08.2023 KR 20230101271; 13.11.2023 KR 20230156500
(71) Applicant: Samsung Electronics Co., Ltd., Suwon-si, Gyeonggi-do 16677 (KR)
(72) Inventor: KIM, Kiwon, Suwon-si, Gyeonggi-do 16677 (KR)
(74) Representative: Appleyard Lees IP LLP
(86) International application number: PCT/KR2024/011136
(87) International publication number: WO 2025/029000

(57) **Abstract**

An electronic device according to the present disclosure may comprise: a housing; a cylinder that protrudes from the housing, and comprises a charging pad that contacts an inner diameter of a wearable device so as to supply power to the wearable device; and a barrier spaced apart from the cylinder and comprising a magnet, wherein the charging pad may comprise a flexible cover that contacts the wearable device, and a power transmission antenna included in the flexible cover. Various other embodiments are possible.

## Description

### [Technical Field]

The disclosure relates to an electronic device for charging a wearable device.

### [Background Art]

Electronic devices including sensors capable of measuring biometric information and/or exercise data (fitness data) while being worn on a user's body have been developed.

Such electronic devices may generally be carried in a pocket or a hand and used while moving, but may also have a form wearable on a part of a user's body or on various structures. An electronic device may be defined as a wearable device in that the electronic device is wearable on the user's body.

The wearable device may include, for example, a watch wearable on the user's wrist or a ring wearable on the user's finger.

The above-described information may be provided as related art for the purpose of assisting in understanding the disclosure. No assertion or decision is made as to whether any of the above might be applicable as prior art with regard to the disclosure.

### [Disclosure of Invention]

### [Technical Problem]

Since a wearable device is worn on a user's body, the wearable device needs to correspond to a user's body size. In order to accommodate various body sizes of users, it is common for wearable devices to be classified and distributed in various sizes even for a single model. For example, a smart ring may be manufactured in various sizes ranging from US size 6 to US size 13, which indicate diameters. Accordingly, there may be a difficulty in producing charging devices in various sizes to charge wearable devices manufactured in various sizes.

An electronic device for charging a wearable device according to the disclosure may provide a cradle for universally charging wearable devices of various sizes.

The electronic device for charging a wearable device according to the disclosure, may allow a power receiving antenna of the wearable device to be aligned with a charging pad by using a magnetic force.

The electronic device for charging a wearable device according to the disclosure may allow the power receiving antenna of the wearable device to be matched by using a flexible charging pad.

The technical problems to be achieved by the disclosure are not limited to those described above, and other technical problems not mentioned herein will be clearly understood by those skilled in the art to which the disclosure pertains.

The electronic device according to the disclosure may include a housing.

The electronic device according to the disclosure may include a cylinder that protrudes from the housing and includes a charging pad configured to contact an inner diameter of the wearable device to supply power to the wearable device.

The electronic device according to the disclosure may include a barrier spaced apart from the cylinder and including a magnet.

The charging pad according to the disclosure may include a flexible cover configured to contact the wearable device.

The charging pad according to the disclosure may include a power transmitting antenna included in the flexible cover.

The electronic device according to the disclosure may supply power to wearable devices by using a magnetic force and a flexible charging pad, without requiring a separate electronic device for charging corresponding to the size of each of differently sized wearable devices.

By supplying power to wearable devices of various sizes using a magnetic force and a flexible charging pad, the electronic device according to the disclosure may reduce manufacturing costs of the electronic device and reduce inventory burden.

Advantageous effects obtainable from the disclosure may not be limited to the above-mentioned effects, and other effects which are not mentioned herein may be clearly understood from the following description by those skilled in the art to which the disclosure pertains.

### [Brief Description of Drawings]

With regard to the description of the drawings, the same or like reference signs may be used to designate the same or like elements.
FIG. 1 is a block diagram of an electronic device in a network environment according to various embodiments of the disclosure.
FIG. 2 is a block diagram of an electronic device according to an embodiment of the disclosure.
FIG. 3 is a view illustrating an electronic device according to an embodiment of the disclosure.
FIG. 4 is a view illustrating at least a portion of the electronic device of FIG. 3, taken along line A-B.
FIG. 5 is a view illustrating a cylinder with at least a portion thereof removed, according to an embodiment of the disclosure.
FIG. 6 is a view illustrating a state in which a first wearable device according to an embodiment of the disclosure is mounted on the electronic device.
FIG. 7 is a view illustrating a state in which a second wearable device according to an embodiment of the disclosure is mounted on the electronic device.
FIG. 8 is a view illustrating the first wearable device and the second wearable device according to an embodiment of the disclosure.
FIG. 9 is a view illustrating configurations of the first wearable device and the second wearable device according to an embodiment of the disclosure.
FIG. 10 is a view illustrating movement of the first wearable device when the first wearable device is mounted on the electronic device.
FIG. 11 is a cross-sectional view of the first wearable device and the electronic device of FIG. 10, taken along line C-D.
FIG. 12 is a view illustrating deformation a charging pad of the electronic device according to an embodiment of the disclosure.
FIG. 13 is a view illustrating an internal region of a cylinder when the charging pad is deformed according to an embodiment of the disclosure.
FIG. 14 is a view schematically illustrating a state in which the first wearable device according to an embodiment of the disclosure is mounted on the electronic device.
FIG. 15 is a view illustrating a state in which the second wearable device according to an embodiment of the disclosure is mounted on the electronic device.
FIG. 16 is a view illustrating an internal region of a cylinder when a charging pad is deformed according to an embodiment of the disclosure.

### [Mode for the Invention]

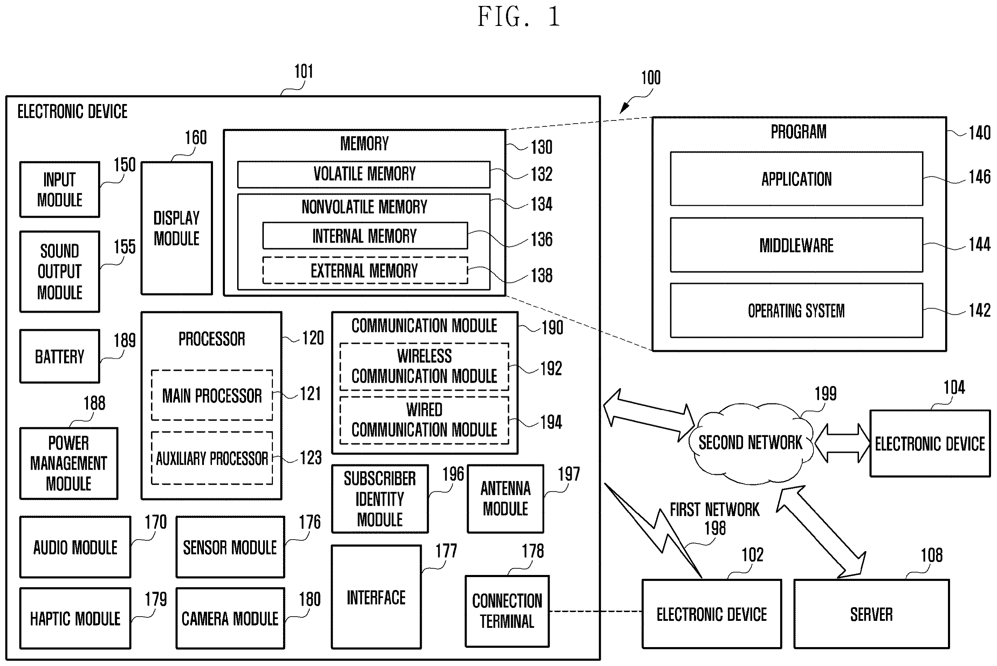

Fig. 1 is a block diagram illustrating an electronic device 101 in a network environment 100 according to various embodiments.

Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

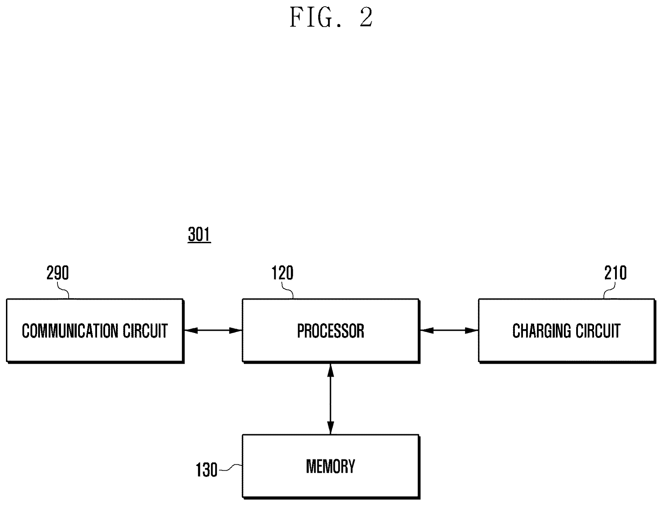

FIG. 2 is a block diagram of an electronic device 301 according to an embodiment of the disclosure.

In an embodiment, the electronic device 301 may include a processor 120, a memory 130, a charging circuit 210, and/or a communication circuit 290.

The electronic device 301 of FIG. 2 may be the same as the electronic device 101 of FIG. 1. However, the disclosure is not limited thereto, and in the electronic device 301 of FIG. 2, at least some components of the electronic device 101 of FIG. 1 may be added or omitted.

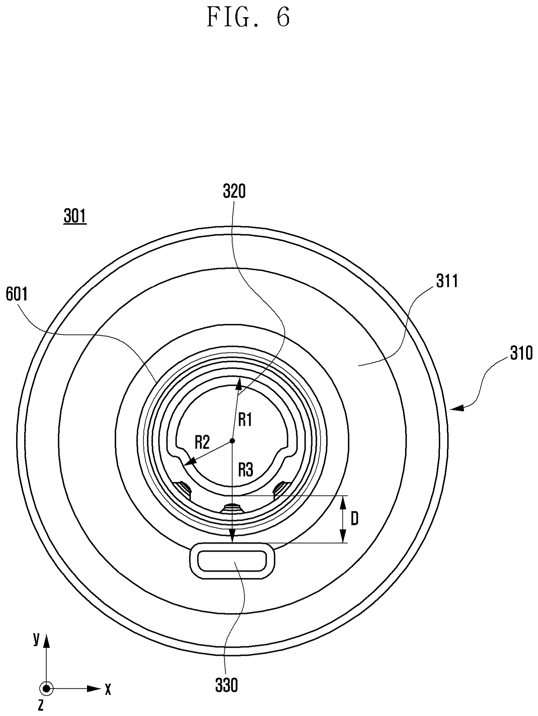

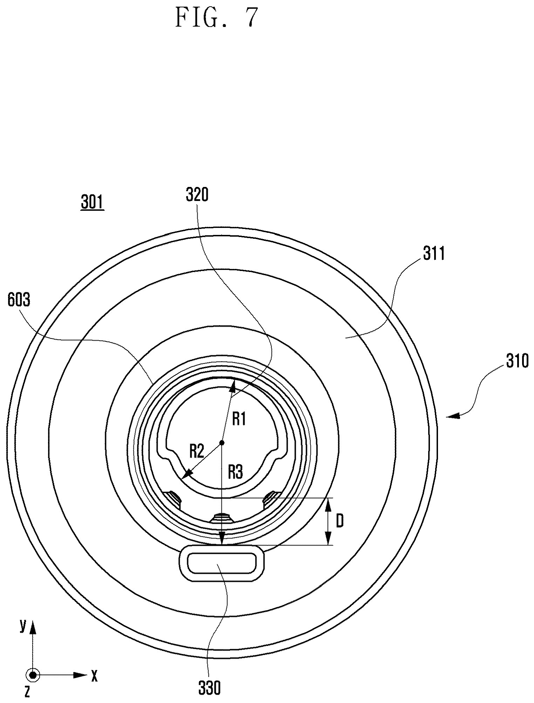

In an embodiment, the processor 120 may control operations of the charging circuit 210 and/or the communication circuit 290. The processor 120 may identify a charging state (e.g., a charging section) of a battery included in a wearable device (e.g., a smart ring, the first wearable device 601 of FIG. 6, or the second wearable device 603 of FIG. 7), and may control a level of power output from the charging circuit 210.

For example, the wearable device (e.g., a smart ring, the first wearable device 601 of FIG. 6, or the second wearable device 603 of FIG. 7) may include a rechargeable battery (e.g., the battery 189 of FIG. 1). A charging section of a battery may include a constant current (CC) section or a constant voltage (CV) section. The charging section of the battery may include a constant current (CC) section in which charging is performed at a predetermined amperage to continuously supply a constant current, or a constant voltage (CV) section in which charging is performed at a predetermined voltage to maintain a constant voltage.

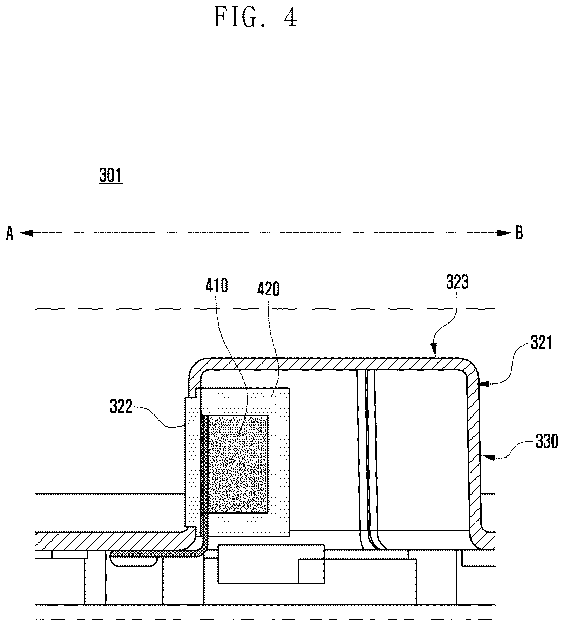

In an embodiment, the charging circuit 210 may, under control of the processor 120, output power through an antenna included in the charging circuit 210 (e.g., the power transmitting antenna 410 of FIG. 4) based on power supplied from an external source.

In an embodiment, the communication circuit 290 may receive information on a charging state (e.g., a charging section) from a wearable device (e.g., a smart ring, the first wearable device 601 of FIG. 6, or the second wearable device 603 of FIG. 7). The electronic device 301 may, under control of the processor 120, control a power level output from the charging circuit 210 based on the received information on the charging state (e.g., the charging section) of the wearable device (e.g., a smart ring, the first wearable device 601 of FIG. 6, or the second wearable device 603 of FIG. 7). The communication circuit 290 of FIG. 2 may be the same as the communication module 190 of FIG. 1.

In an embodiment, the memory 130 may store in advance information on a power level corresponding to a charging state (e.g., a charging section) of a battery included in a wearable device (e.g., a smart ring, the first wearable device 601 of FIG. 6, or the second wearable device 603 of FIG. 7). The electronic device 301 may control the power level output from the charging circuit 210 based on the information stored in the memory 130 on the power level corresponding to the charging state (e.g., the charging section) of the battery included in the wearable device (e.g., a smart ring, the first wearable device 601 of FIG. 6, or the second wearable device 603 of FIG. 7).

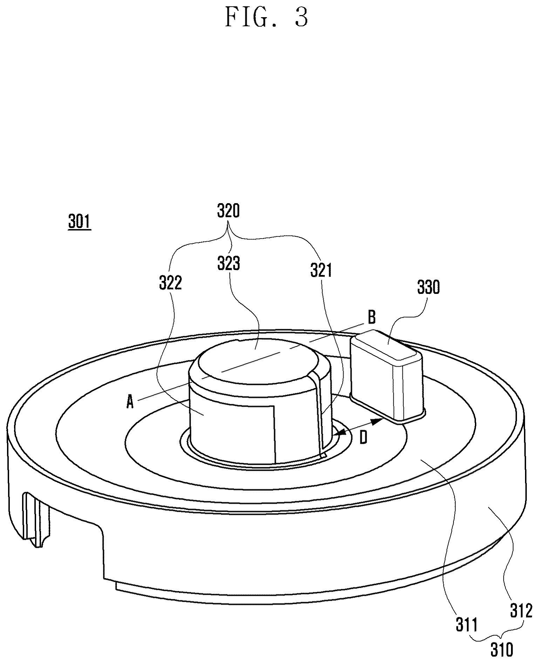

FIG. 3 is a view illustrating an electronic device 301 according to an embodiment of the disclosure.

FIG. 4 is a view illustrating at least a portion of the electronic device 301 of FIG. 3, taken along line A-B.

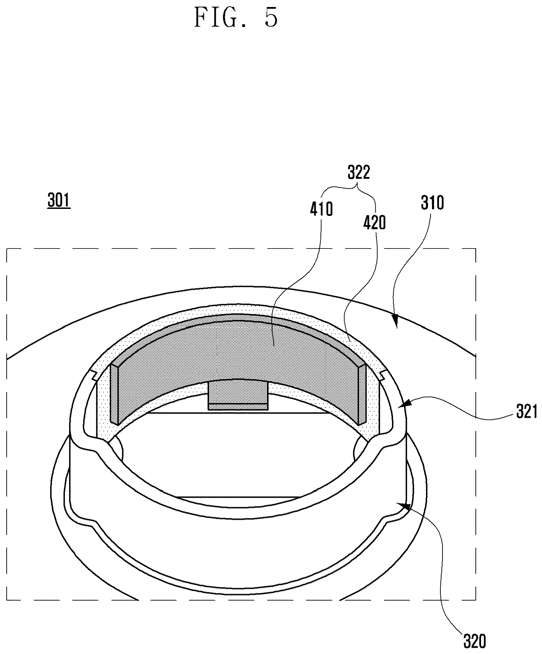

FIG. 5 is a view illustrating a cylinder 320 with at least a portion thereof removed, according to an embodiment of the disclosure.

Referring to FIGS. 3 to 5, the electronic device 301 may include a housing 310, a cylinder 320, and/or a barrier 330.

According to an embodiment, the electronic device 301 may be a charging cradle capable of supplying power to a wearable device (e.g., a smart ring, the first wearable device 601 of FIG. 6, or the second wearable device 603 of FIG. 7) to charge a battery of the wearable device (e.g., a smart ring, the first wearable device 601 of FIG. 6, or the second wearable device 603 of FIG. 7).

According to an embodiment, the housing 301 may include a cylinder 320 and/or a barrier 330. The housing 301 may include a substantially flat disk 311 and a side surface member 312 surrounding the disk 311. The disk 311 and the side surface member 312 may form a space. A processor 120, a memory 130, a charging circuit 210, and/or a communication circuit 290 may be mounted in the space formed by the disk 311 and the side surface member 312.

According to an embodiment, the cylinder 320 and the barrier 330 may be disposed on at least a portion of the disk 311. The cylinder 320 and the barrier 330 may have shapes protruding, at least in part, from a plane of the disk 311.

According to an embodiment, the cylinder 320 may be in contact, at least in part, with an inner diameter surface of a wearable device (e.g., a smart ring, the first wearable device 601 of FIG. 6, or the second wearable device 603 of FIG. 7). The cylinder 320 may include, at least in part, a charging pad 322.

According to an embodiment, the cylinder 320 may include a support member 321, a charging pad 322, and/or a lid 323.

According to an embodiment, the cylinder 320 may include a rigid region and a flexible region. The rigid region may include the support member 321 and/or the lid 323. The flexible region may include the charging pad 322.

According to an embodiment, the cylinder 320 may protrude from the housing 310. For example, the cylinder 320 may protrude from the housing 310 by a certain height.

In an embodiment, the support member 321 may be in contact, at least in part, with an inner diameter surface of a wearable device (e.g., a smart ring, the first wearable device 601 of FIG. 6, or the second wearable device 603 of FIG. 7). When the wearable device (e.g., a smart ring, the first wearable device 601 of FIG. 6, or the second wearable device 603 of FIG. 7) is mounted on the electronic device 301, the support member 321 may support at least a portion of the wearable device (e.g., a smart ring, the first wearable device 601 of FIG. 6, or the second wearable device 603 of FIG. 7).

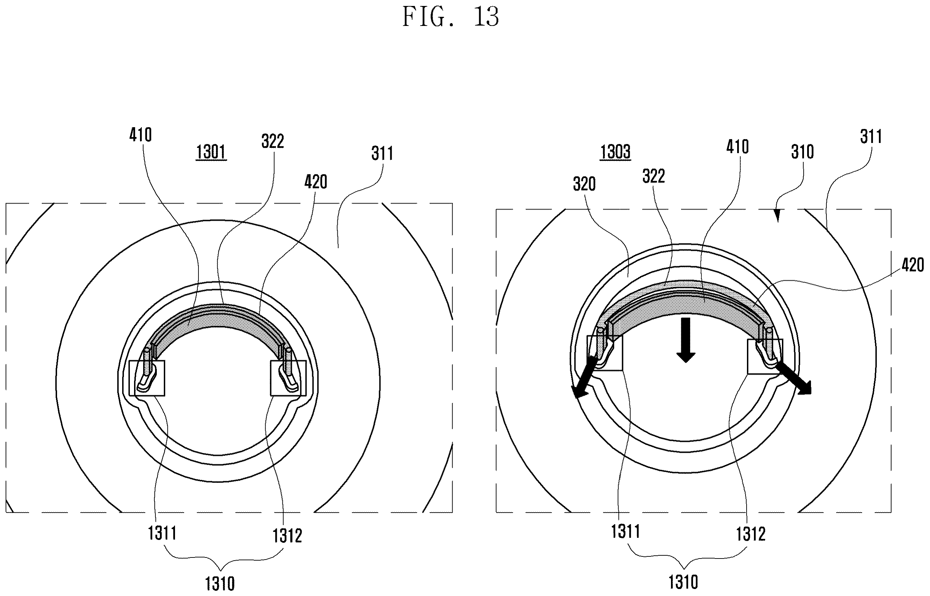

According to an embodiment, the support member 321 may form an internal space of the cylinder 320 together with the lid 323. When the flexible charging pad 322 moves, the cylinder 320 may include, in the internal space, at least one stopper (e.g., the stoppers 1311 and 1312 of FIG. 13) configured to restrict movement of the charging pad 322.

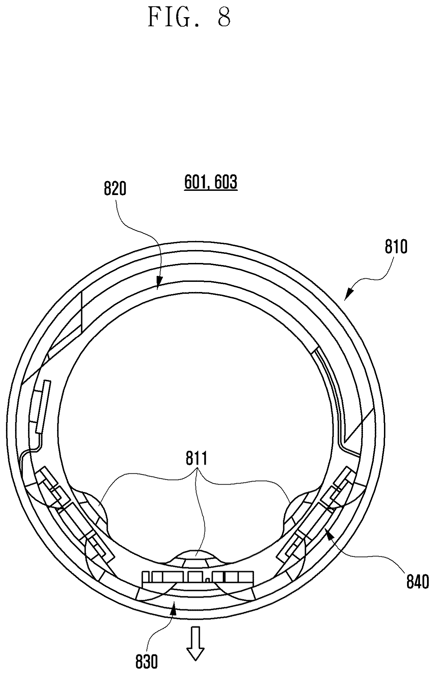

According to an embodiment, the charging pad 322 may correspond, at least in part, to a power receiving antenna (e.g., the power receiving antenna 820 of FIG. 8) of a wearable device (e.g., a smart ring, the first wearable device 601 of FIG. 6, or the second wearable device 603 of FIG. 7).

According to an embodiment, the charging pad 322 may include a power transmitting antenna 410 and/or a flexible cover 420.

According to an embodiment, the charging pad 322 may be made of the same material as the flexible cover 420. However, the disclosure is not limited thereto, and the charging pad 322 and the flexible cover 420 may be made of different materials from each other.

According to an embodiment, the power transmitting antenna 410 may be a flexible printed circuit board.

According to an embodiment, the power transmitting antenna 410 may be electrically connected, at least in part, to the charging circuit 210.

According to an embodiment, the flexible cover 420 may correspond, at least in part, to the power receiving antenna (e.g., the power receiving antenna 820 of FIG. 8) of the wearable device (e.g., a smart ring, the first wearable device 601 of FIG. 6, or the second wearable device 603 of FIG. 7).

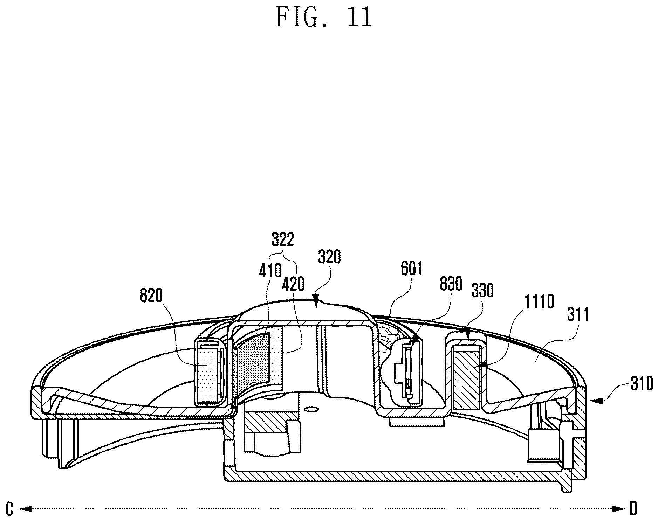

According to an embodiment, when a wearable device (e.g., a smart ring, the first wearable device 601 of FIG. 6, or the second wearable device 603 of FIG. 7) is mounted on the cylinder 320 and is attracted by a magnet (e.g., the magnet 1110 of FIG. 11) included in the barrier 330, the flexible cover 420 may be deformed at least in part. When at least a portion of the flexible cover 420 is deformed, a power receiving antenna (e.g., the power receiving antenna 820 of FIG. 8) and the power transmitting antenna 410 may be matched. For example, the matching may be a state in which the power receiving antenna (e.g., the power receiving antenna 820 of FIG. 8) and the power transmitting antenna 410 are arranged in parallel. The flexible cover 420 may be made of a flexible material. For example, the flexible cover 420 may include silicone, rubber, or polyurethane, and may include a composite material in which silicone, rubber, or polyurethane are combined.

According to an embodiment, when the wearable device (e.g., a smart ring, the first wearable device 601 of FIG. 6, or the second wearable device 603 of FIG. 7) is mounted on the cylinder 320 and is rotated by a magnet (e.g., the magnet 1110 of FIG. 11) included in the barrier 330, the power receiving antenna (e.g., the power receiving antenna 820 of FIG. 8) and the power transmitting antenna 410 may be aligned. For example, the alignment may be a state in which the power receiving antenna (e.g., the power receiving antenna 820 of FIG. 8) and the power transmitting antenna 410 correspond to each other.

According to an embodiment, the wearable device (e.g., a smart ring, the first wearable device 601 of FIG. 6, or the second wearable device 603 of FIG. 7) may include a magnetic member (e.g., the magnetic member 830 of FIG. 8) configured to respond to a magnet (e.g., the magnet 1110 of FIG. 11).

According to an embodiment, the barrier 330 may protrude from the housing 310. For example, the barrier 330 may protrude from the housing 310 by a certain height. Substantially, the barrier 330 and the cylinder 320 may protrude from the housing 310 by substantially the same height. The barrier 330 may have a certain width.

According to an embodiment, the barrier 330 may include, therein, a magnet (e.g., the magnet 1110 of FIG. 11). When the wearable device (e.g., a smart ring, the first wearable device 601 of FIG. 6, or the second wearable device 603 of FIG. 7) is mounted on the cylinder 320, the magnet (e.g., the magnet 1110 of FIG. 11) may rotate the wearable device or pull the wearable device toward the barrier 330 based on a magnetic force. When the wearable device (e.g., a smart ring, the first wearable device 601 of FIG. 6, or the second wearable device 603 of FIG. 7) is rotated or pulled toward the barrier 330 by the magnet (e.g., the magnet 1110 of FIG. 11), a power receiving antenna (e.g., the power receiving antenna 820 of FIG. 8) and the power transmitting antenna 410 may be aligned, or the power receiving antenna (e.g., the power receiving antenna 820 of FIG. 8) and the power transmitting antenna 410 may be matched.

According to an embodiment, the barrier 330 may be spaced apart from the cylinder 320 by a certain distance D. The certain distance D may be designed based on a maximum size among various sizes of wearable devices (e.g., a smart ring, the first wearable device 601 of FIG. 6, or the second wearable device 603 of FIG. 7).

FIG. 6 is a view illustrating a state in which a first wearable device 601 according to an embodiment of the disclosure is mounted on the electronic device 301.

FIG. 7 is a view illustrating a state in which a second wearable device 603 according to an embodiment of the disclosure is mounted on the electronic device 301.

Referring to FIGS. 6 and 7, the cylinder 320 may include a region having an outer radius corresponding to a first radius R1 and a region having an outer radius corresponding to a second radius R2. The second radius R2 may be less than or equal to the first radius R1. The cylinder 320 may include regions having different outer diameters so as to correspond to at least one sensor included in the wearable device (e.g., a heat rate monitor (HRM) sensor 811 of FIG. 8). However, the disclosure is not limited thereto, and the cylinder 320 may have a uniform outer diameter.

According to an embodiment, the first wearable device 601 and the second wearable device 603 may have different sizes from each other. For example, the wearable devices may be manufactured in various sizes ranging from US size 6 to US size 13, which indicate diameter sizes. Among wearable devices under production, the first wearable device 601 may have the smallest size in terms of an internal radius. Among wearable devices under production, the second wearable device 603 may have the largest size in terms of an internal radius.

According to an embodiment, the internal radius of the first wearable device 601 may correspond to the first radius R1 of the cylinder 320. The internal radius of the first wearable device 601 may be equal to the first radius R1 of the cylinder 320. The internal radius of the first wearable device 601 may be greater than the first radius R1 of the cylinder 320.

According to an embodiment, the internal radius of the second wearable device 603 may correspond to a third radius R3. The third radius R3 may correspond to a sum of the second radius R2 of the cylinder 320 and the certain distance D. The third radius R3 may be greater than the sum of the second radius R2 of the cylinder 320 and the certain distance D.

According to an embodiment, the inner diameter surface of each of the first wearable device 601 and the second wearable device 603 may be in contact, at least in part, with the cylinder 320 protruding from the disk 311 of the housing 310.

FIG. 8 is a view illustrating the first wearable device 601 and the second wearable device 603 according to an embodiment of the disclosure.

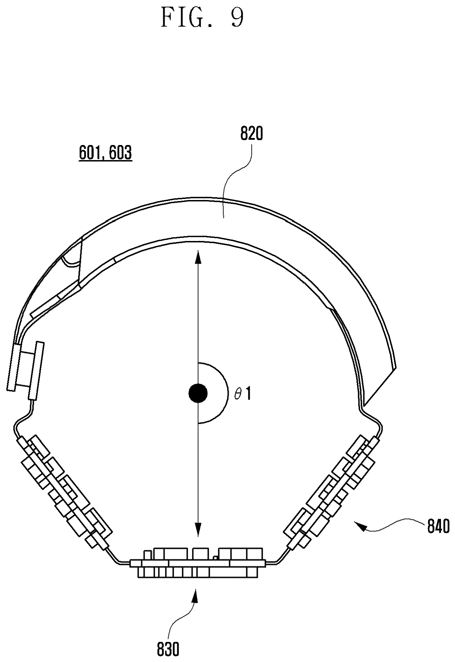

FIG. 9 is a view illustrating configurations of the first wearable device 601 and the second wearable device 603 according to an embodiment of the disclosure.

Referring to FIGS. 8 and 9, the first wearable device 601 and the second wearable device 603 may each include a ring-shaped housing 810.

According to an embodiment, the ring-shaped housing 810 may include therein a power receiving antenna 820, at least one HRM sensor 811, a magnetic member 830, and/or a circuit board 840.

According to an embodiment, the power receiving antenna 820 and/or the at least one HRM sensor 811 may be disposed on the circuit board 840 of each of the first wearable device 601 and the second wearable device 603. The power receiving antenna 820 and the at least one HRM sensor 811 may protrude to the outside of the ring-shaped housing 810. However, the disclosure is not limited thereto, and the first wearable device 601 and the second wearable device 603 may further include a processor (e.g., the processor 120 of FIG. 1), a memory (e.g., the memory 130 of FIG. 1), a communication circuit (e.g., the communication module 190 of FIG. 1), a battery (e.g., the battery 189 of FIG. 1), and/or an antenna (e.g., the antenna 197 of FIG. 1). The processor (e.g., the processor 120 of FIG. 1), the memory (e.g., the memory 130 of FIG. 1), the communication circuit (e.g., the communication module 190 of FIG. 1), the battery (e.g., the battery 189 of FIG. 1), and/or the antenna (e.g., the antenna 197 of FIG. 1) may be disposed on the circuit board 840.

According to an embodiment, the power receiving antenna 820 may receive power from the charging pad 322 of the electronic device 301. The received power may be stored in a battery (e.g., the battery 189 of FIG. 1) of each of the first wearable device 601 and the second wearable device 603.

According to an embodiment, the magnetic member 830 may be made of a paramagnetic material or a magnetic material (e.g., a ferromagnetic material).

According to an embodiment, the magnetic member 830 and the power receiving antenna 820 may form a certain angle θ1. The certain angle θ1 may be, for example, about 180 degrees. The magnetic member 830 and the power receiving antenna 820 may be arranged to be substantially parallel to each other.

According to an embodiment, the barrier 330 and the charging pad 322 may be arranged at the same angle as the certain angle θ1 of FIG. 9. The barrier 330 and the charging pad 322 may be arranged to be substantially parallel to each other.

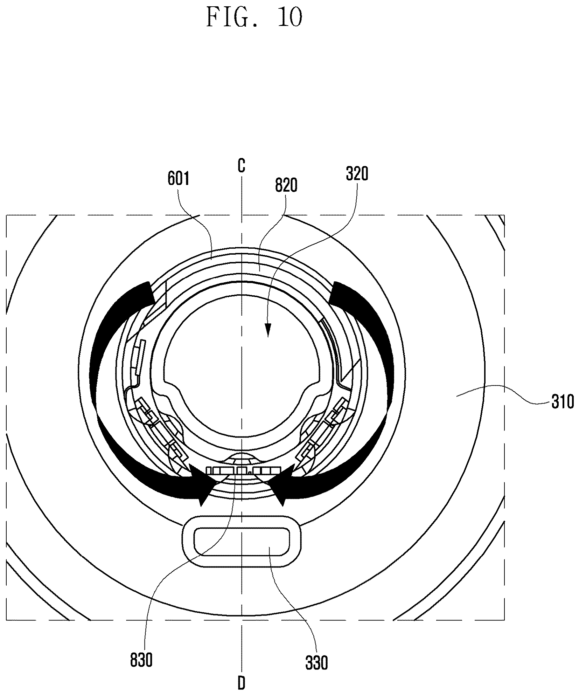

FIG. 10 is a view illustrating movement of the first wearable device 601 when the first wearable device 601 is mounted on the electronic device 301.

FIG. 11 is a cross-sectional view of the first wearable device 601 and the electronic device 301 of FIG. 10, taken along line C-D.

Referring to FIGS. 10 and 11, an inner diameter surface of the first wearable device 601 may be in contact, at least in part, with the cylinder 320 protruding from the disk 311 of the housing 310.

According to an embodiment, the barrier 330 may include therein a magnet (e.g., the magnet 1110 of FIG. 11). When the first wearable device 601 is mounted on the cylinder 320, the magnetic member 830 may rotate toward the magnet 1110. In this case, the first wearable device 601 may rotate toward the barrier 330 by the magnetic member 830 and the magnet 1110.

According to an embodiment, when the first wearable device 601 is mounted on the cylinder 320, the magnetic member 830 may be pulled toward the magnet 1110. In this case, the first wearable device 601 may be pulled toward the barrier 330 by the magnetic member 830 and the magnet 1110.

According to an embodiment, when the first wearable device 601 is rotated or pulled toward the barrier 330 by the magnetic member 830 and the magnet 1110, the power receiving antenna 820 and the power transmitting antenna 410 may be aligned, or the power receiving antenna 820 and the power transmitting antenna 410 may be matched.

According to an embodiment, the flexible cover 420 may be deformed so as to correspond to the inner diameter surface of the first wearable device 601.

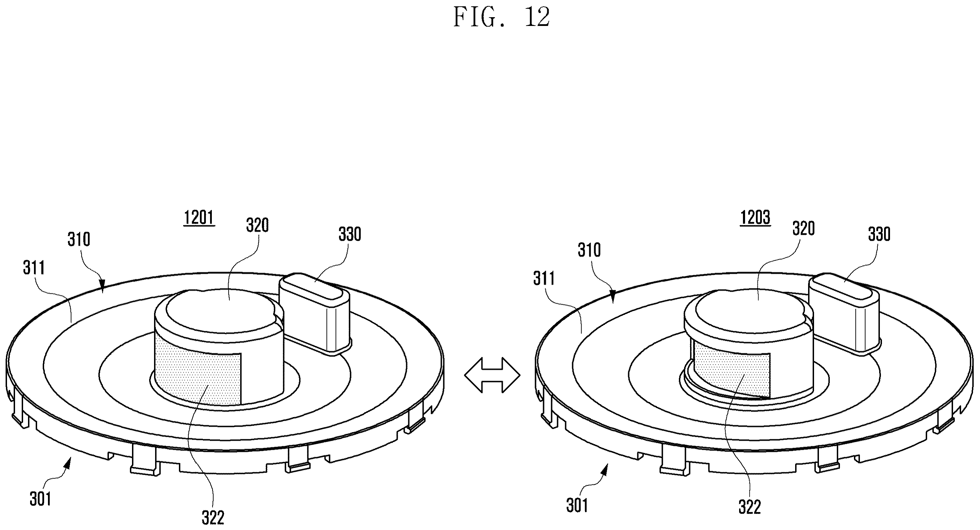

FIG. 12 is a view illustrating deformation of the charging pad 322 of the electronic device 301 according to an embodiment of the disclosure.

FIG. 13 is a view illustrating an internal region of the cylinder 320 when the charging pad 322 is deformed according to an embodiment of the disclosure.

Reference numerals 1201 and 1301 indicate states of the charging pad 322 before wearable devices (the first wearable device 601 of FIG. 6 and the second wearable device 603 of FIG. 7) are mounted on the electronic device 301. Reference numerals 1203 and 1303 indicate states of the charging pad 322 when the wearable devices (the first wearable device 601 of FIG. 6 and the second wearable device 603 of FIG. 7) are mounted on the electronic device 301. In FIG. 12, descriptions of components of the electronic device 301 that have been described above may be omitted.

According to an embodiment, the charging pad 322 may be flexible, and thus may be deformed so as to correspond to the inner diameter surfaces of the wearable devices (the first wearable device 601 of FIG. 6 and the second wearable device 603 of FIG. 7).

According to an embodiment, the cylinder 320 may include at least one stopper 1310 in the internal space thereof.

According to an embodiment, when the charging pad 322 moves, the at least one stopper 1310 may restrict the movement of the charging pad 322.

According to an embodiment, the at least one stopper 1310 may include a first stopper 1311 and a second stopper 1312. The first stopper 1311 and the second stopper 1312 may support at least a portion of the charging pad 322.

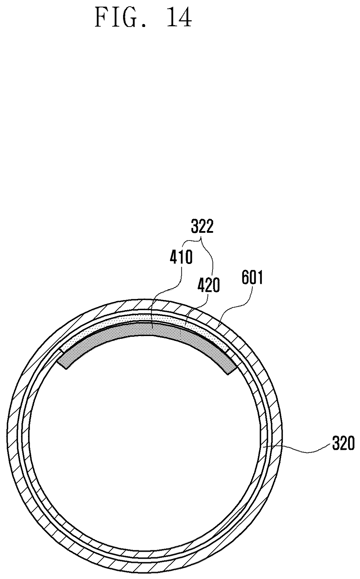

FIG. 14 is a view schematically illustrating a state in which the first wearable device 601 according to an embodiment of the disclosure is mounted on the electronic device 301.

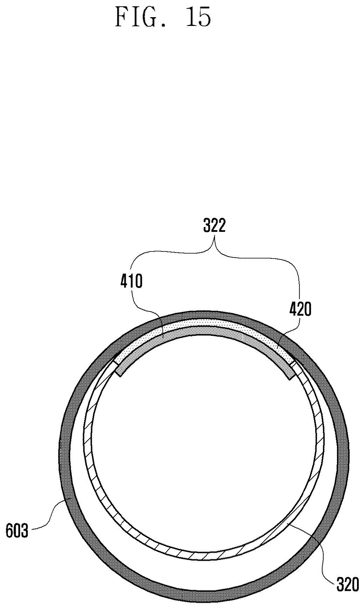

FIG. 15 is a view schematically illustrating a state in which the second wearable device 603 according to an embodiment of the disclosure is mounted on the electronic device 301.

Referring to FIGS. 14 and 15, when the first wearable device 601 is seated on the cylinder 320, the inner diameter surface of the first wearable device 601 may be in contact, at least in part, with the charging pad 322 of the cylinder 320. When the second wearable device 603 is seated on the cylinder 320, the inner diameter surface of the second wearable device 603 may be in contact, at least in part, with the charging pad 322 of the cylinder 320. A region corresponding to the inner diameter surface of the first wearable device 601 that is in contact with the charging pad 322 may include a power receiving antenna (e.g., the power receiving antenna 820 of FIG. 8). Accordingly, when the first wearable device 601 or the second wearable device 603 is seated on the cylinder 320, the power receiving antenna 820 may be aligned and/or matched with the power transmitting antenna 410 of the electronic device 301.

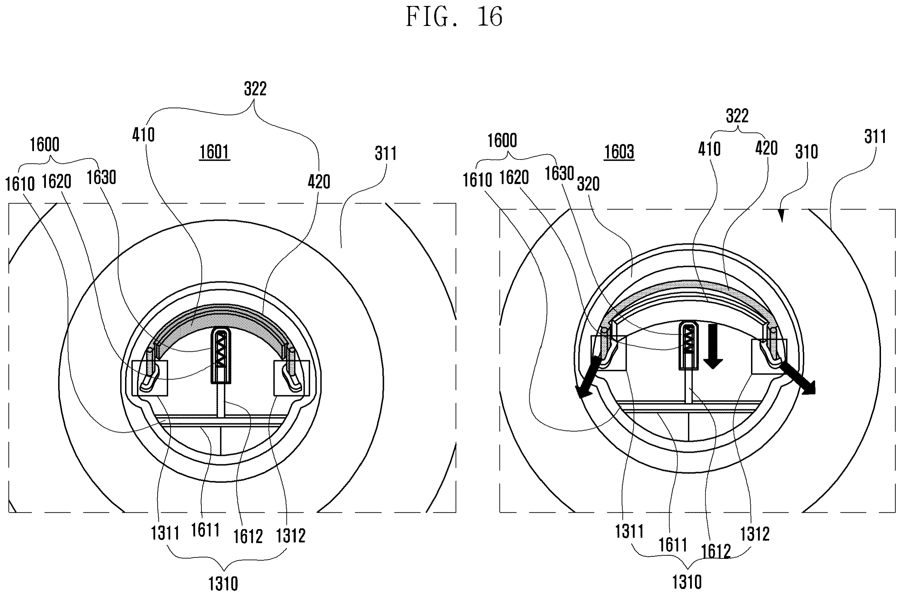

FIG. 16 is a view illustrating an internal region of the cylinder 320 when the charging pad 322 is deformed according to an embodiment of the disclosure.

Reference numerals 1201 of FIG. 12 and 1601 indicate the states of the charging pad 322 before wearable devices (e.g., the first wearable device 601 of FIG. 6 and the second wearable device 603 of FIG. 7) are mounted on the electronic device 301. Reference numerals 1203 of FIG. 12 and 1603 indicate the states of the charging pad 322 when the wearable devices (e.g., the first wearable device 601 of FIG. 6 and the second wearable device 603 of FIG. 7) are mounted on the electronic device 301. In FIG. 16, descriptions of components of the electronic device 301 that have been described above may be omitted.

According to an embodiment, the charging pad 322 may be flexible, and thus may be deformed so as to correspond to the inner diameter surfaces of the wearable devices (e.g., the first wearable device 601 of FIG. 6 and the second wearable device 603 of FIG. 7).

According to an embodiment, when the charging pad 322 moves, the cylinder 320 may include, in the internal space thereof, at least one stopper 1310 and a position restoration guide structure 1600.

According to an embodiment, when the charging pad 322 moves, the at least one stopper 1310 and the position restoration guide structure 1600 may restrict movement of the charging pad 322.

According to an embodiment, the position restoration guide structure 1600 may support at least a portion of the charging pad 322. The position restoration guide structure 1600 may provide an elastic force so as to allow the charging pad 322 to quickly return to its original position when the wearable devices (e.g., the first wearable device 601 of FIG. 6 and the second wearable device 603 of FIG. 7) are removed after being mounted on the electronic device 301.

According to an embodiment, the position restoration guide structure 1600 may include a support structure 1610, an elastic element 1620, and an elastic element cover 1630. The position restoration guide structure 1600 may include a first structure 1611 extending across an interior of the cylinder 320 and a second structure 1612 oriented toward the charging pad 322 at a certain angle (e.g., about 90 degrees) with respect to the first structure 1611.

According to an embodiment, the position restoration guide structure 1600 may be coupled, at least in part, to the elastic element 1620 and the elastic element cover 1630. The elastic element 1620 may be a spring. When the elastic element 1620 moves, the elastic element cover 1630 may move together with the elastic element 1620, thereby preventing the elastic element 1620 from being separated from the position restoration guide structure 1600.

According to an embodiment, the electronic device 301 may include a housing 310, a cylinder 320 protruding from the housing 310 and including a charging pad 322 configured to contact the inner diameter of a wearable device (e.g., the first wearable device 601 of FIG. 6 or the second wearable device 603 of FIG. 7) to supply power to the wearable device, and a barrier 330 spaced apart from the cylinder 320 and including a magnet (e.g., the magnet 1110 of FIG. 11). According to an embodiment, the charging pad 322 may include a flexible cover 420 configured to contact the wearable device (e.g., the first wearable device 601 of FIG. 6 or the second wearable device 603 of FIG. 7), and a power transmitting antenna 410 included in the flexible cover 420.

According to an embodiment, the charging pad 322 and the barrier 330 may be arranged in parallel.

According to an embodiment, the power transmitting antenna 410 may include a flexible printed circuit board.

According to an embodiment, the cylinder 320 may include a stopper 1310 configured to support movement of the charging pad 322, or a position restoration guide structure 1600 configured to restore a position of the charging pad 322.

According to an embodiment, the cylinder 320 may have a first region and a second region having different outer diameters.

According to an embodiment, the barrier 330 may protrude from the housing 310.

According to an embodiment, the charging pad 322 may be flexible by virtue of the flexible cover 420 and the power transmitting antenna 410.

According to an embodiment, the curvature of the charging pad 322 may correspond to the curvature of the cylinder 320.

According to an embodiment, the sum of the outer radius of the cylinder 320 and a certain distance may correspond to a radius of a wearable device (e.g., the first wearable device 601 of FIG. 6 or the second wearable device 603 of FIG. 7).

According to an embodiment, the outer radius of the cylinder 320 may correspond to the radius of the wearable device (e.g., the first wearable device 601 of FIG. 6 or the second wearable device 603 of FIG. 7).

According to an embodiment, the housing 310 may have, at least in part, a flat surface. According to an embodiment, the housing 310 may include the cylinder 320 and the barrier 330 on the flat surface.

According to an embodiment, the electronic device 301 may further include a processor 120. According to an embodiment, the processor 120 may control transmission power of the power transmitting antenna 410.

According to an embodiment, the electronic device 301 may further include a communication circuit 290. According to an embodiment, the communication circuit 290 may receive information on a charging state from the wearable device (e.g., the first wearable device 601 of FIG. 6 or the second wearable device 603 of FIG. 7).

According to an embodiment, the electronic device 301 may further include a memory 130.

According to an embodiment, the memory 130 may store information related to transmission power according to a charging state of a wearable device (e.g., the first wearable device 601 of FIG. 6 or the second wearable device 603 of FIG. 7).

According to an embodiment, the wearable device (e.g., the first wearable device 601 of FIG. 6 or the second wearable device 603 of FIG. 7) may include a ring-shaped housing corresponding to the shape of the cylinder 320.

According to an embodiment, the ring-shaped housing may include a power receiving antenna corresponding to the charging pad 322, a battery configured to store power received from the power receiving antenna, and a magnetic member (e.g., the magnetic member 830 of FIG. 8) corresponding to a magnet (e.g., the magnet 1110 of FIG. 11).

According to an embodiment, the magnet (e.g., the magnet 1110 of FIG. 11) may react with the magnetic member (e.g., the magnetic member 830 of FIG. 8) by a magnetic force to rotate the wearable device (e.g., the first wearable device 601 of FIG. 6 or the second wearable device 603 of FIG. 7), thereby aligning the power receiving antenna (e.g., the power receiving antenna 820 of FIG. 8) and the charging pad 322 so as to correspond in position.

According to an embodiment, the magnet (e.g., the magnet 1110 of FIG. 11) may react with the magnetic member (e.g., the magnetic member 830 of FIG. 8) by a magnetic force to pull the wearable device (e.g., the first wearable device 601 of FIG. 6 or the second wearable device 603 of FIG. 7) toward the magnet, thereby aligning the power receiving antenna (e.g., the power receiving antenna 820 of FIG. 8) and the charging pad 322 in parallel.

According to an embodiment, the power receiving antenna (e.g., the power receiving antenna 820 of FIG. 8) and the magnetic member (e.g., the magnetic member 830 of FIG. 8) may be arranged in parallel.

According to an embodiment, a wearable device (e.g., the first wearable device 601 of FIG. 6 or the second wearable device 603 of FIG. 7) may include a circuit board including a power receiving antenna (e.g., the power receiving antenna 820 of FIG. 8), a battery, and a magnetic member (e.g., the magnetic member 830 of FIG. 8).

According to an embodiment, the wearable device (e.g., the first wearable device 601 of FIG. 6 or the second wearable device 603 of FIG. 7) may include at least one HRM sensor, a near-field communication (NFC) circuit, and a temperature sensor.

The electronic device according to various embodiments set forth herein may be one of various types of electronic devices. The electronic device may include, for example, a portable communication device (e.g., a smart phone), a computer device, a portable multimedia device, a portable medical device, a camera, a wearable device, or a home appliance. The electronic device according to embodiments of the disclosure is not limited to those described above.

It should be appreciated that the embodiments and the terms used therein are not intended to limit the technological features set forth herein to particular embodiments and the disclosure includes various changes, equivalents, or alternatives for a corresponding embodiment. With regard to the description of the drawings, similar reference numerals may be used to designate similar or relevant elements. A singular form of a noun corresponding to an item may include one or more of the items, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one or all possible combinations of the items enumerated together in a corresponding one of the phrases. Such terms as "a first," "a second," "the first," and "the second" may be used to simply distinguish a corresponding element from another, and does not limit the elements in other aspect (e.g., importance or order). If an element (e.g., a first element) is referred to, with or without the term "operatively" or "communicatively", as "coupled with/to" or "connected with/to" another element (e.g., a second element), it means that the element may be coupled/connected with/to the other element directly (e.g., wiredly), wirelessly, or via a third element.

As used in various embodiments of the disclosure, the term "module" may include a unit implemented in hardware, software, or firmware, and may be interchangeably used with other terms, for example, "logic," "logic block," "component," or "circuit". The "module" may be a single integrated component, or a minimum unit or part thereof, adapted to perform one or more functions. For example, according to an embodiment, the "module" may be implemented in the form of an application-specific integrated circuit (ASIC).

Various embodiments as set forth herein may be implemented as software (e.g., the program 140) including one or more instructions that are stored in a storage medium (e.g., the internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it. This allows the machine to be operated to perform at least one function according to the at least one instruction invoked. The one or more instructions may include codes generated by a compiler or code executable by an interpreter. The machine-readable storage medium may be provided in the form of a non-transitory storage medium. Herein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, methods according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play Store TM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each element (e.g., module or program) of the above-described elements may include a single entity or multiple entities, and some of the multiple entities may be separately disposed in another element. According to various embodiments, one or more of the above-described elements or operations may be omitted, or one or more other elements or operations may be added. Alternatively or additionally, a plurality of elements (e.g., modules or programs) may be integrated into a single element. In such a case, according to various embodiments, the integrated element may still perform one or more functions of each of the plurality of elements in the same or similar manner as they are performed by a corresponding one of the plurality of elements before the integration. According to various embodiments, operations performed by the module, the program, or another element may be carried out sequentially, in parallel, repeatedly, or heuristically, or one or more of the operations may be executed in a different order or omitted, or one or more other operations may be added.

## Claims

1. An electronic device comprising:
a housing;
a cylinder protruding from the housing and comprising a charging pad configured to contact an inner diameter of a wearable device so as to supply power to the wearable device; and
a barrier spaced apart from the cylinder and including a magnet,
wherein the charging pad comprises
a flexible cover (420) configured to contact the wearable device, and
a power transmitting antenna (410) included in the flexible cover.

2. The electronic device of claim 1, wherein the charging pad and the barrier are arranged in parallel.

3. The electronic device of claim 1, wherein the power transmitting antenna comprises a flexible printed circuit board.

4. The electronic device of claim 1, wherein the cylinder comprises:
a stopper configured to support movement of the charging pad; or
a position restoration guide structure configured to restore a position of the charging pad.

5. The electronic device of claim 1, wherein the charging pad is flexible by the flexible cover and the power transmitting antenna.

6. The electronic device of claim 1, wherein the cylinder has a first region and a second region having different outer diameters.

7. The electronic device of claim 1, wherein the barrier protrudes from the housing.

8. The electronic device of claim 1, wherein a curvature of the charging pad corresponds to a curvature of the cylinder.

9. The electronic device of claim 1, wherein a sum of an outer radius of the cylinder and a certain distance corresponds to a radius of the wearable device.

10. The electronic device of claim 1, wherein an outer radius of the cylinder corresponds to a radius of the wearable device.

11. The electronic device of claim 1, wherein the housing has a flat surface formed on at least a part thereof, and comprises the cylinder and the barrier on the flat surface.

12. The electronic device of claim 1, further comprising:
a processor,
wherein the processor is configured to control transmission power of the power transmitting antenna.

13. The electronic device of claim 1, further comprising:
a communication circuit,
wherein the communication circuit is configured to receive information on a charging state from the wearable device.

14. The electronic device of claim 1, further comprising:
a memory,
wherein the memory stores information on transmission power according to a charging state of the wearable device.

15. The electronic device of claim 2, wherein the wearable device comprises a ring-shaped housing corresponding to a shape of the cylinder,
wherein the ring-shaped housing comprises:
a power receiving antenna corresponding to the charging pad,
a battery configured to store power received from the power receiving antenna, and
a magnetic member corresponding to the magnet.
